# EUROPEAN PATENT APPLICATION

(11) **EP 4 524 545 A1**
(43) Date of publication of application: **19.03.2025**
(21) Application number: 23803570.3
(22) Date of filing: 10.05.2023
(51) Int. Cl.: G01N 1/04, C12N 1/02, C12N 15/11, G01N 1/12

(54) **METHOD FOR SEPARATING TARGET SUBSTANCE CONTAINED IN TARGET LIQUID**

(30) Priority: 11.05.2022 JP 2022078243
(71) Applicant: Craif Inc., Tokyo 113-0034 (JP)
(72) Inventor: YASUI, Takao, Nagoya-shi, Aichi 464-8601 (JP); YOKOI, Akira, Nagoya-shi, Aichi 464-8601 (JP)
(74) Representative: Michalski Hüttermann & Partner Patentanwälte mbB
(86) International application number: PCT/JP2023/017525
(87) International publication number: WO 2023/219095

(57) **Abstract**

An aspect of the present disclosure provides a method for preserving a target substance in a target liquid. The method includes bringing a fiber sheet into contact with a target liquid to absorb the target liquid into the fiber sheet; drying the fiber sheet; and washing the fiber sheet. Thus, for example, the target substance can be simply or efficiently separated from contaminants. Thus, for example, the target substance can be simply separated.

## Description

### TECHNICAL FIELD

The present disclosure relates to a method for separating a target substance contained in a target liquid.

### BACKGROUND ART

Selective capture or separation of a substance, such as a biomolecule, present in a liquid is important for examining, adjusting, or treating the properties of the target substance.

For example, extracellular vesicles (exosomes; hereinafter sometimes referred to as "EVs"), which are one of biomolecules, have been found to induce cancer metastasis as one of their in vivo functions and are attracting attention.

An extracellular vesicle is an endoplasmic reticulum membrane with a size of approximately 40 nm to 1000 nm secreted from a cell in a living body and is present in a body fluid, such as blood, urine, saliva, or semen. A membrane protein, an adhesion molecule, an enzyme, or the like derived from a secretory cell is present on the surface of the extracellular vesicle, and a nucleic acid, such as mRNA or miRNA, is contained in the extracellular vesicle. Thus, it has been found that the extracellular vesicle affects a recipient cell by being transmitted to and taken up by another cell.

To selectively obtain a target substance, various methods have been developed; for example, a physical method, such as ultracentrifugation, a chemical method, such as an aggregation reagent method, or a capturing method using a ZnO nanowire device or the like. However, for example, to capture, separate, or analyze a biomolecule present in the body, it is necessary to obtain a certain volume of tumor, body fluid, or the like.

As another example, it is known to capture extracellular vesicles (EVs) using cellulose nanofibers (Patent Literature 1). However, this literature discloses a device for adsorbing EVs and discloses that the spaces (nanopores) of cellulose nanofibers should have a certain size distribution, but does not disclose a method for physically preventing EVs once captured from being released again.

In either case, there has been no method for collecting a biomolecule, such as EV, from such a trace amount of body fluid that can wet a surface of an organ, skin, or the like.

### CITATION LIST

### PATENT LITERATURE

PTL 1: WO 2020/090859 A1

### SUMMARY OF INVENTION

An aspect of the present disclosure provides a method for preserving a target substance in a target liquid. The method includes bringing a fiber sheet into contact with a target liquid to absorb the target liquid into the fiber sheet; drying the fiber sheet; and washing the fiber sheet. Thus, for example, the target substance can be simply or efficiently separated from contaminants. Thus, for example, the target substance can be simply separated.

Another aspect of the present disclosure provides a method for separating a target substance in a target liquid. The method includes bringing a fiber sheet into contact with a target liquid to absorb the target liquid into the fiber sheet; drying the fiber sheet; washing the fiber sheet; and applying an aqueous solution to the fiber sheet after drying to release a captured target substance from the fiber sheet. Thus, for example, the target substance can be simply or efficiently separated from contaminants.

Additional aspects and advantages of the present disclosure will become readily apparent to those skilled in the art from the following detailed description, wherein only exemplary embodiments of the present disclosure are shown and described. As will be understood, in the present disclosure, another different embodiment is possible, and its several details can be modified in various obvious respects without departing from the present disclosure. Thus, the drawings and description are to be essentially regarded as illustrative and not as restrictive.

### BRIEF DESCRIPTION OF DRAWINGS

[Fig. 1] Fig. 1 is a flow chart of a method for separating a target substance in a target liquid according to an embodiment of the present disclosure.
[Fig. 2] Fig. 2 is a flow chart of a method for separating a target substance in a target liquid according to an embodiment of the present disclosure.
[Fig. 3] Fig. 3 is a series of CG images generated for steps of a method for separating a target substance in a target liquid according to an embodiment of the present disclosure.
[Fig. 4] Fig. 4 is a series of SEM images taken at each step of a method for separating a target substance in a target liquid according to an embodiment of the present disclosure.
[Fig. 5] Fig. 5 is a graph of the porous rate of a fiber sheet measured for each step of a method for separating a target substance in a target liquid according to an embodiment of the present disclosure.
[Fig. 6] Fig. 6 is a flow chart of a method for preserving a target substance in a target liquid according to an embodiment of the present disclosure.
[Fig. 7] Fig. 7 is a CG image created for part of steps of a method for separating a target substance in a target liquid according to an embodiment of the present disclosure.
[Fig. 8] Fig. 8 shows, as an example of the present disclosure, a correlation between miRNAs obtained in two experiments performed using a method for separating a target substance in a target liquid.
[Fig. 9] Fig. 9 shows annotation rates of synthetic miRNAs intentionally introduced as impurities into miRNAs obtained using a method for separating a target substance in a target liquid.
[Fig. 10] Fig. 10 shows the relationship between the drying time of a fiber sheet and the annotation rate of miRNAs of separated EVs in one example.
[Fig. 11] Fig. 11(a) shows, as an example of the present disclosure, the size distribution of EVs separated from human serum using a method for separating a target substance in a target liquid, and Fig. 11(b) shows the total amount thereof.
[Fig. 12] Fig. 12 shows, as an example of the present disclosure, the expression levels of membrane proteins of EVs separated from human serum using a method for separating a target substance in a target liquid.
[Fig. 13] Fig. 13(a) shows, as an example of the present disclosure, the size distribution of EVs separated from mouse ascites using a method for separating a target substance in a target liquid, and Fig. 13(b) shows the total amount thereof.
[Fig. 14] Fig. 14 shows, as an example of the present disclosure, the expression levels of membrane proteins of EVs separated from mouse ascites using a method for separating a target substance in a target liquid.
[Fig. 15] Fig. 15 is, as an example of the present disclosure, a heat map of miRNAs obtained from the pelvic peritoneum and the liver surface of a mouse using a method for separating a target substance in a target liquid.
[Fig. 16] Fig. 16 is, as an example of the present disclosure, a heat map of miRNAs obtained from a mouse tumor surface and a surface near the tumor and, for comparison purposes, miRNAs obtained from a mouse tumor tissue and a tissue near the tumor, using a method for separating a target substance in a target liquid.
[Fig. 17] Fig. 17 is the results of PCA analysis of mouse miRNAs shown in the heat map above.
[Fig. 18] Fig. 18 is, as an example of the present disclosure, a heat map of the expression of miRNAs contained in EVs obtained using a fiber sheet from the pelvic peritoneum and the liver surface at day 0 and day 4.
[Fig. 19] Fig. 19 is, as an example of the present disclosure, a PCA map of the expression of miRNAs in EVs and tissues obtained using a fiber sheet.
[Fig. 20] Fig. 20 shows annotation rates of miRNAs obtained from a tumor surface and the surface near the tumor of a human ovarian cancer patient shown in the heat map above.
[Fig. 21] Fig. 21 is, as an example of the present disclosure, a heat map of the expression of miRNAs obtained by RNA sequencing for miRNAs from EVs and tumors obtained using a fiber sheet.
[Fig. 22] Fig. 22 is, as an example of the present disclosure, a PCA map of miRNAs obtained using a fiber sheet from each organ or body fluid.
[Fig. 23] Fig. 23 shows, as an example of the present disclosure, the results of PCA analysis of various data.

### Description of Embodiments

### <First Embodiment: Separation of Target>

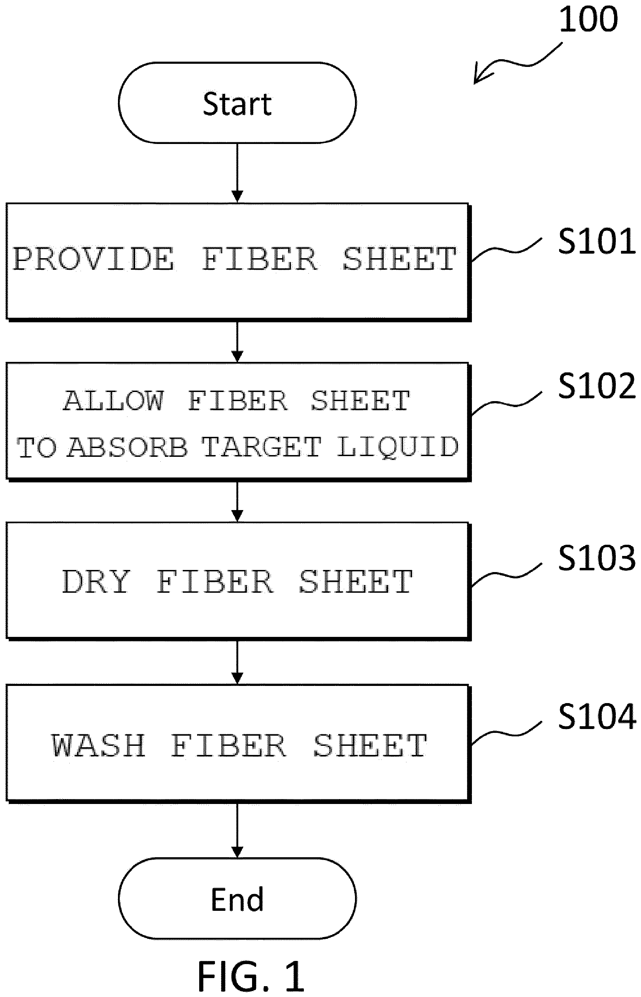

Fig. 1 is a flow chart 100 of a method for separating a target substance in a target liquid according to an embodiment of the present disclosure. A fiber sheet is provided (S101). A target liquid containing a target substance is brought into contact with the fiber sheet to absorb the target liquid into the fiber sheet (S 102). The fiber sheet is then dried (S103). In some embodiments, the fiber sheet may be transported or stored in a dried state. Subsequently, the fiber sheet may be washed.

### <Second Embodiment: Separation of Target>

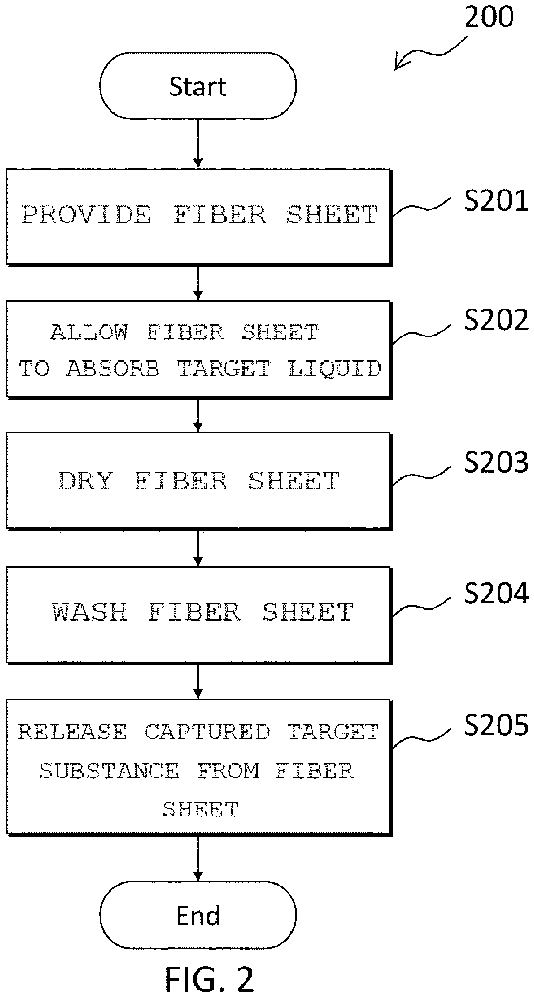

Fig. 2 is a flow chart 200 of a method for separating a target substance in a target liquid according to an embodiment of the present disclosure. A fiber sheet is provided (S201). A target liquid containing a target substance is brought into contact with the fiber sheet to absorb the target liquid into the fiber sheet (S202). The fiber sheet is then dried (S203). The dried fiber sheet is washed to remove impurities while the fiber sheet retains the target substance (S204). The captured target substance is released from the fiber sheet (S205).

### <Fiber Sheet>

The term "fiber sheet", as used herein, refers generally to a flat member with pores formed by mechanically and/or chemically processing fibers. The fiber sheet includes a fabric, a non-woven fabric, a fabric, paper, or the like. A fiber sheet according to the present disclosure preferably has hydrophilicity to such an extent that an aqueous solution permeates into the inside due to capillarity or higher hydrophilicity.

**In** some embodiments, the fibers may be chemical fibers, natural fibers, or fibers produced by mixing a plurality of types of fibers.

**In** some embodiments, the natural fibers may be plant fibers, such as cellulose, cotton, hemp, or linen. **In** some embodiments, the fibers may be animal fibers, such as wool, silk, or cashmere.

The term "cellulose fiber sheet", as used herein, refers generally to a fiber sheet composed mainly of or containing cellulose fibers. The term "cellulose fibers", as used herein, refers to fibers composed mainly of cellulose. The cellulose fibers may be cellulose nanofibers (CNF). Cellulose nanofibers typically have a diameter of a few nanometers (nm) to tens of nanometers. In many instances, by way of a non-limiting example, cellulose nanofibers have a width of approximately 3 nm to 100 nm.

Cellulose fibers can be, for example, chemically and/or mechanically obtained from wood pulp or another raw material. A typical method for producing cellulose nanofibers is described below. First, wood fibers (cellulose fibers) are taken out from wood chips and are pulped. The cellulose fibers are composed of a bundle of innumerable cellulose nanofibers. Next, the cellulose fibers are collided with each other in the presence of a TEMPO catalyst in a solvent at a high pressure to loosen the bundle of cellulose fibers. Thus, cellulose nanofibers can be obtained. The methods for producing cellulose fibers and a cellulose fiber sheet described above are non-limiting examples, and another production method may be adopted.

A solvent containing the cellulose nanofibers is subjected to suction filtration to aggregate the cellulose nanofibers by surface tension or to form a film. The cellulose cellulose nanofiber solvent may be water or the like. **In** a certain embodiment, the film may be a non-woven fabric.

For nanopores, a liquid with low surface tension (hereinafter also referred to as a "low-surface-tension solvent") is applied to wet cellulose nanofibers aggregated by suction filtration. This is followed by suction, and the solvent contained in the mass of the aggregated cellulose nanofibers is replaced with a low-surface-tension solvent or dried. Thus, nanopores are formed inside the assembly of cellulose nanofibers.

The size of the nanopores can be adjusted, for example, by the applied low-surface-tension solvent. The surface tension of the low-surface-tension solvent may be lower than the surface tension of water (20°C, 72.75 mN/m) and may be in the range in which nanopores can be formed. For example, the surface tension of the solvent at 20°C may be less than or equal to 35 mN/m, 30 mN/m, 25 mN/m, 20 mN/m, or the like. The low-surface-tension solvent is, for example, but not limited to, tertiary butyl alcohol (20.7 mN/m), ethanol (22.55 mN/m), isopropanol (20.8 mN/m), or the like.

The methods for forming nanopores and adjusting the size of the nanopores are examples, and another method may be used. For example, high-pressure processing conditions may be employed to loosen cellulose fibers. For example, the type of pulp may be changed. For example, cellulose derived from another source (for example, a microorganism, such as an acetic acid bacterium (the genus Acetobacter or the like); or an animal, such as an ascidian) may be used. In this way, the width of cellulose nanofibers may be changed to adjust the size of nanopores.

In some embodiments, the fiber sheet may be formed of cellulose fibers (pulp). Cellulose fibers (pulp) can be dispersed in a solvent to produce a fiber sheet in almost the same manner as the cellulose nanofibers.

The space (pore size) of cellulose fibers can be adjusted in almost the same manner as in the cellulose nanofibers. Cellulose fibers often have a width (diameter) in the range of approximately 20 µm to 40 µm. Thus, the size of the space may be several nanometers to several micrometers, approximately 10 nm to approximately 1000 nm, or approximately 1 µm to 100 µm.

In some embodiments, the fibers may be chemical fibers. In some embodiments, the chemical fibers may be polymer fibers or synthetic fibers of poly(vinyl alcohol) (PVA or the like). The chemical fibers may also be polyester fibers, such as poly(ethylene terephthalate), poly(naphthalene terephthalate), poly(ethylene naphthalate), or poly(trimethylene terephthalate); polyamide fibers, such as nylon; acrylic fibers, such as acrylonitrile; polyolefin fibers, such as polyethylene or polypropylene; cellulose regenerated fibers, such as rayon, cupra, or Polynosic; cellulose-based semi-synthetic fibers, such as acetate; protein-based semi-synthetic fibers, such as promix; polyurethane fibers; vinylon fibers; glass fibers; carbon fibers (including carbon nanotube), or the like.

The term "pore size" or "characteristic size", as used herein, refers to the size of pores of a fiber sheet. The pore size may be 2 nm, 3 nm, 4 nm, 5 nm, 6 nm, 7 nm, 8 nm, 9 nm, 10 nm, 15 nm, 20 nm, 30 nm, or more. The pore size may be 20 nm, 30 nm, 40 nm, 50 nm, 60 nm, 70 nm, 80 nm, 90 nm, 100 nm, 150 nm, 200 nm, 300 nm, 400 nm, 500 nm, or less. The pore size may range from approximately 4 nm to approximately 200 nm, approximately 40 nm to approximately 200 nm, approximately 200 nm to approximately 500 nm, approximately 4 nm to approximately 100 nm, approximately 5 nm to approximately 90 nm, or approximately 10 nm to approximately 80 nm.

For example, a fiber sheet with a pore size in the range of approximately 40 nm to approximately 200 nm is suitable for capturing small EVs with a similar size. For example, a fiber sheet with a pore size in the range of approximately 200 nm to approximately 500 nm is suitable for capturing large EVs with a similar size. These are examples, and a fiber sheet with another pore size may be used to capture EVs with a similar size.

A fiber sheet according to the present disclosure preferably shrinks by drying. In other words, evaporation of the absorbed solvent (aqueous solution or non-aqueous solution) reduces the pore size of the fiber sheet. The fiber sheet is preferably produced such that the pore size in a wet state is greater than or equal to the size of the target to be captured and after drying is smaller than the size of the target to be captured.

The pore size can be measured before use, in the dry state, after washing, or after desorption of a substance to be captured. The pore size can be measured by a mercury intrusion method, electron microscopy, or the like, or a combination thereof. Unlike a porous solid material, it may be difficult to uniquely define the space between fibers. The mercury intrusion method is believed to reflect the pore size or inter-fiber distance of a fiber sheet but does not necessarily give these values. Thus, the results of the mercury intrusion method may be configured by another method (for example, microscopy) or may be considered as a measure of pore size.

The pore size may be defined or determined based on the measurement of the target substance (for example, EVs) or the measurement of a substance associated therewith (for example, an EV-containing nucleic acid) or characteristics thereof. The characteristics of a fiber sheet or the production conditions of the fiber sheet that provide the best measurement results or predetermined results may be defined as characteristics exchangeable with the pore size.

The pore size may alternatively be defined by, for example, the amount of EVs separated by a fiber sheet, the pore size of EVs that gives a peak, the distribution or range of sizes of EVs, the amount or profile of a nucleic acid contained in EVs separated by a fiber sheet, or the like, or may be expressed using them as measures.

In some embodiments, the fiber sheet may be configured to substantially allow a target object to enter or pass through the space of the fiber sheet during production or when absorbing a target liquid and to substantially retain a target in the fiber sheet and/or prevent the target from flowing out during washing (after drying or before washing).

### <Target Liquid>

The "target liquid" includes a liquid containing a target substance, a liquid that is considered to contain a target substance, or a liquid that does not contain a target substance but is used for the purpose of separating a target substance or for a purpose related thereto.

The target liquid (also referred to as a target solution) may be a body fluid or a liquid derived from a body fluid (such as a diluted liquid or a treated liquid). The solution may be a solution that is not a body fluid (derived from a non-body fluid), an artificially prepared liquid, or a liquid mixture of a body fluid or a solution derived from a body fluid and a solution derived from a non-body fluid. The solution may be a solution used for sample measurement or a solution used for calibration measurement. It may be used as a solution, a stock solution, or a diluted or concentrated liquid of a stock solution. The solution may be a standard liquid or a calibration liquid. A sample to be measured may be a specimen. The solution may contain a physiological buffer solution, such as phosphate-buffered saline (PBS) or an N-tris(hydroxymethyl)methyl-2-aminoethanesulfonic acid buffer solution (TES), containing a substance to be collected. The body fluid may contain an additive agent. For example, a stabilizer or a pH adjuster may be added to the additive agent.

The body fluid may be, but is not limited to, blood, serum, plasma, or lymph, tissue fluid, such as intertissue fluid, intercellular fluid, or interstitial fluid, or coelomic fluid, serous cavity fluid, pleural effusion, ascites, pericardial fluid, cerebrospinal fluid (spinal fluid), joint fluid (synovial fluid), or aqueous humor (hydatoid). The body fluid may be digestive juice, such as saliva, gastric juice, bile, pancreatic juice, or intestinal juice, or may be sweat, tears, nasal mucus, urine, semen, vaginal fluid, amniotic fluid, or milk.

The body fluid may be a human body fluid. The body fluid may be an animal body fluid. The animal may be a reptile, a mammal, or an amphibian. The mammal may be a dog, cat, cow, horse, sheep, pig, hamster, mouse, squirrel, or primate, such as monkey, gorilla, chimpanzee, bonobo, or human.

### <Target Substance>

The target substance may be a biomolecule. The biomolecule may be an organelle or a vesicle. The vesicle may be, but is not limited to, a vacuole, a lysosome, a transport vesicle, secretion, a gas vesicle, an extracellular matrix vesicle, an extracellular vesicle, or the like, and can include two or more of them. The extracellular vesicle (EV) may be, but is not limited to, exosome, an exosome complex, ectosome, a shedding microvesicle, a microvesicle, a membrane particle, a plasma membrane, an apoptotic bleb, or the like.

The biomolecule may be or include, without limitation, a cell. The cell may be an erythrocyte, a leukocyte, an immune cell, or the like. The biomolecule may be a virus, a bacterium, or the like.

The target substance may contain a nucleic acid.

The nucleic acid may be or may contain ribonucleic acid (RNA). The RNA may be, but is not limited to, messenger RNA (mRNA), transfer RNA (tRNA), ribosomal RNA (rRNA), non-coding RNA (ncRNA), microRNA (miRNA), ribozyme, double-stranded RNA (dsRNA), or the like, or may include two or more of them. The RNA may be modified. The RNA or miRNA may be involved in the onset or progression of cancer, a cardiovascular disease, a neurodegenerative disease, a psychiatric disorder, a chronic inflammatory disease, or the like. The miRNA may be an RNA of a type that promotes or positively regulates canceration (onco miRNA (oncogenic miRNA, cancer-promoting miRNA)) or may be an RNA of a type that suppresses or negatively regulates canceration (Tumor Suppressor miRNA (cancer-suppressing miRNA)).

The nucleic acid may be or contain deoxyribonucleic acid (DNA).

### <Absorption>

A target liquid can be absorbed into a fiber sheet. The target liquid is absorbed into the fiber sheet by coming into contact with at least part of the fiber sheet. The target liquid may be absorbed into the fiber sheet by surface tension. The target liquid may also be absorbed into the fiber sheet by pressure.

In some embodiments, the fiber sheet may be brought into contact with the surface of a target liquid that is macroscopically present in a liquid state. In some embodiments, the fiber sheet may be immersed in a target liquid. For example, the fiber sheet can be brought into contact with collected urine or immersed in urine to absorb the urine.

In some embodiments, the fiber sheet may be brought into contact with a target liquid on a solid surface (the surface of a target portion on which a target substance is present; hereinafter also referred to as a "target surface"). The target surface may be in a humid environment. In such a case, the fiber sheet may be attached to the solid surface. For example, the fiber sheet may be brought into contact with the surface of a tissue or an organ of an organism (hereinafter referred to simply as an organ). The organism may be a plant or an animal. The organ of the organism may be a biological organ, such as skin, a mucosa, or an internal organ. The surface of the organ of the organism may be non-invasively accessed, may be invasively accessed by a surgical technique or the like, or may be minimally invasively accessed, for example, by endoscopic surgery. The surface may be the surface of the organ or an exposed surface of an interior that is first made accessible through an incision or the like.

Animal organs include the circulatory system, the digestive system, the endocrine system, the urinary system, the lymphatic system, the integumentary system, the nervous system, the reproductive system, the respiratory system, and the motor system (including skeletons and muscles). However, the methods of use and classification of these terms are examples, and others may be employed.

For example, the fiber sheet can be attached to the surface of an internal organ, such as a liver, which is exposed during surgical operation or is removed from the body by surgery, endoscopic surgery, or the like, to absorb a body fluid on the surface.

In some embodiments, the fiber sheet may be applied to or brought into contact with the skin or mucosa to absorb a body fluid on the surface of the skin or mucosa. When the amount of body fluid on the body surface is insufficient, a solution may be dropped on the body surface to promote absorption into the fiber sheet.

In some embodiments, the fiber sheet may be attached to or brought into contact with a portion that is not normally visible from the outside, such as the armpit (or axilla), the oral cavity (such as the tongue), the nasal cavity, the esophagus, the stomach, the intestine, or the vagina, to absorb a body fluid present or secreted on the surface of the skin or the mucosa.

In some embodiments, a body fluid may be temporarily collected, and the fiber sheet may be brought into contact with the body fluid to absorb the body fluid at a location away from the subject. The body fluid includes, for example, urine, tears, saliva, gastric juice, vaginal fluid, semen, blood, ascites, pleural effusion, or the like.

### <Drying>

In some embodiments, the fiber sheet is dried after absorbing a target liquid. The drying may be performed at room temperature, at a temperature higher than room temperature or by heating, or at a temperature lower than room temperature. The temperature may be set such that the subsequent measurement of a target substance is stably performed or such that the measurement is not substantially adversely affected. The drying can be performed at the humidity at the climatic conditions of the place and time or at a lower regulated humidity. The drying is not completed if the drying time is too short. The process is not efficient if the drying time is too long. Thus, an appropriate drying time may be determined.

### <Control of Pore Size>

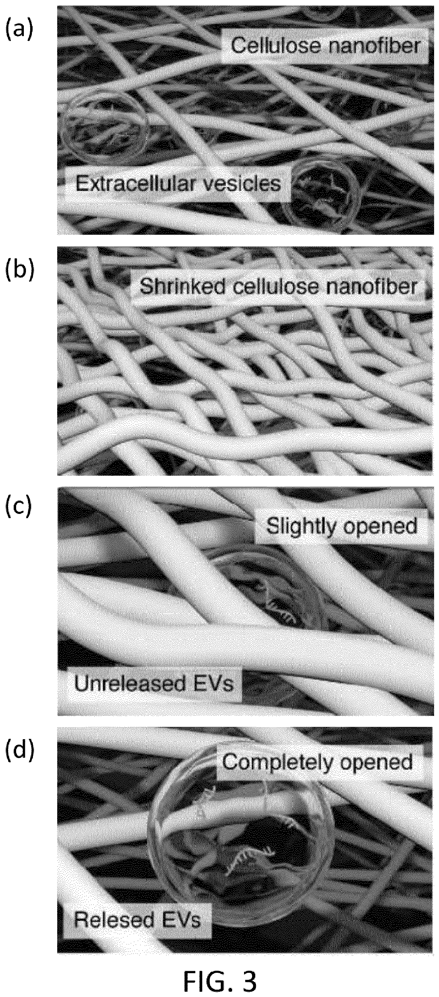

Fig. 3 shows, by computer graphics (CG), each step of using a cellulose nanofiber (CNF) sheet as a fiber sheet to capture extracellular vesicles as a target substance, separating them from contaminants, and then releasing them from the fiber sheet.

As shown in Fig. 3(a), extracellular vesicles enter the CNF sheet by contact with a target liquid. Substances larger than the pore size of the CNF sheet cannot enter the sheet. Next, drying reduces the pore size of the CNF sheet (Fig. 3(b)). Thus, the internal extracellular vesicles are captured by the CNF and cannot exit again to the outside. Due to contact with an aqueous solution, washing slightly increases the pore size of the CNF sheet. However, washing under appropriate conditions does not excessively increase the pore size of the CNF sheet, and the extracellular vesicles remain captured inside the CNF sheet and cannot flow out to the outside (Fig. 3(c)). Substances smaller than the pore size (for example, contaminants) can be washed out from the CNF sheet. Under other solution conditions, the pore size of the CNF sheet can be sufficiently increased to take out the extracellular vesicles from the CNF sheet (Fig. 3(d)).

Thus, by the method according to some embodiments described in the present disclosure, a substance (target substance) with a pore size smaller than the initial pore size and larger than the pore size at the time of washing can be separated from a substance (contaminants) with another pore size and obtained. In other words, a fiber sheet with one or both of the two pore sizes corresponding to the size of a target substance can be selected to selectively separate and/or obtain the target substance.

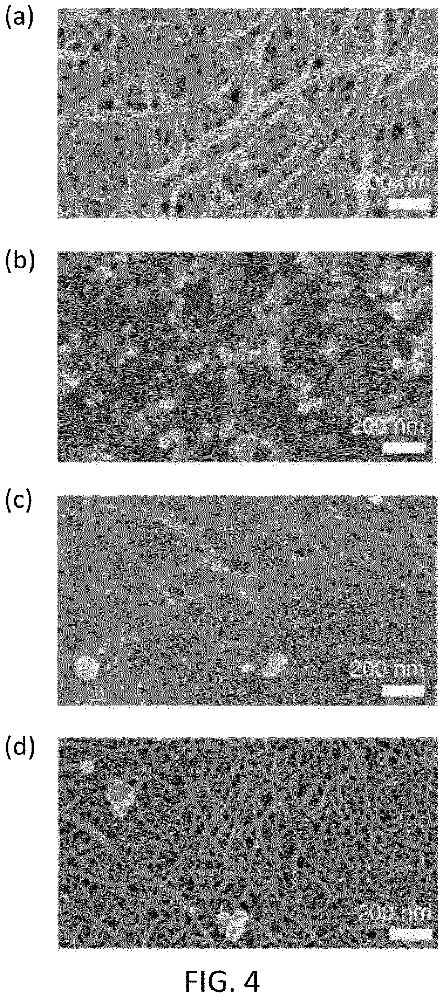

Figs. 4(a) to 4(d) show a series of SEM images of the surface of the CNF sheet corresponding to each step of Figs. 3(a) to 3(d). Human saliva was used as a target liquid, and the CNF sheet was produced to have a pore size capable of selectively capturing extracellular vesicles.

In Fig. 4(a), the surface structure of the initial CNF sheet could be observed. As shown in Fig. 4(b), a large number of extracellular vesicles were captured by the CNF sheet after contact with the target liquid. After washing (Fig. 4(c)), the pore size of the CNF sheet decreases, and the internal structure is not clearly visible, but it is known from the subsequent experiment that extracellular vesicles are captured inside. Under other solution conditions, the pore size of the CNF sheet could be sufficiently increased to take out the extracellular vesicles from the CNF sheet. Consequently, the extracellular vesicles were rarely observed (Fig. 4(d)).

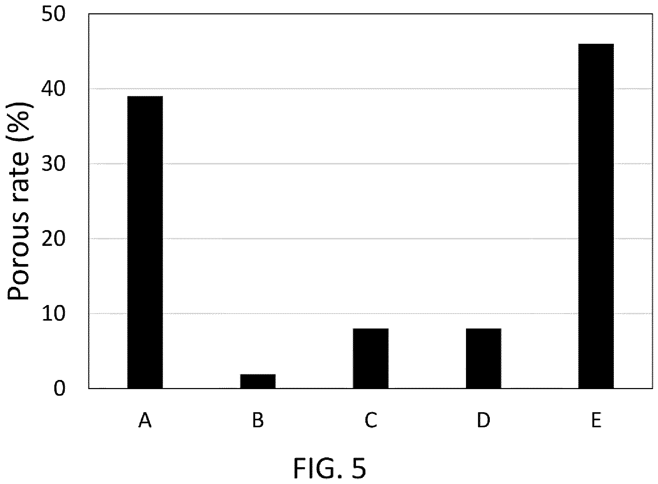

The pore size of the CNF sheet at each step was measured by the mercury intrusion method. Fig. 5 shows the porous rate (%) defined by the mercury intrusion method. A to E indicate the following states. A: unused state after production, B: dried state after dropping saliva, C: washed state with PBS for 10 seconds, D: washed with PBS for 10 seconds and treated with a lysis buffer for 5 minutes, and E: washed state with PBS for 5 minutes to sufficiently open the pore and take out the captured substance.

In B to E, a tBuOH treatment was performed before the measurement by the mercury intrusion method. More specifically, the sheet was immersed in tBuOH immediately after each step. The sheet was removed after 1 hour, was dried at room temperature, and was analyzed. To perform the mercury intrusion method, the CNF sheet must be dried. Without any treatment, there is a risk of the pores shrinking again due to drying. The tBuOH treatment suppresses the reduction in pore size that may be caused by subsequent drying, and enables more accurate measurement of the pore size.

### <Third Embodiment: On-Site Disruption of Target>

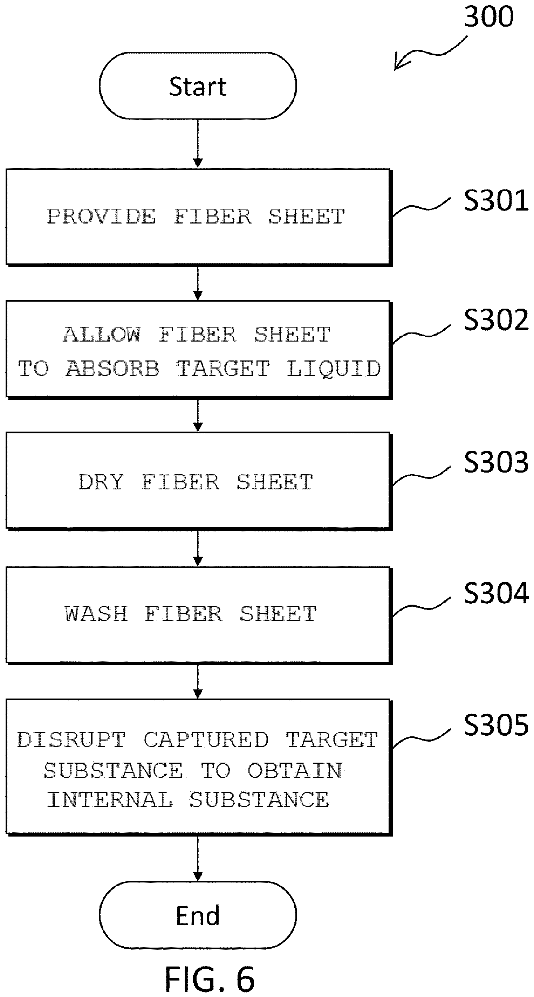

Fig. 6 is a flow chart 300 of a method for separating a target substance in a target liquid according to an embodiment of the present disclosure. A fiber sheet is provided (S301). A target liquid containing a target substance is brought into contact with the fiber sheet to absorb the target liquid into the fiber sheet (S302). The fiber sheet is then dried (S303). The dried fiber sheet is washed to remove impurities while retaining the target substance in the fiber sheet (S304). The target substance captured by the fiber sheet is disrupted to obtain an internal substance of the target substance (S305).

In some embodiments, the target substance may not necessarily be disrupted within the fiber sheet to obtain the internal substance. In some embodiments, the method for separating the target substance in the target liquid may include disrupting the target substance captured by the fiber sheet instead of S305.

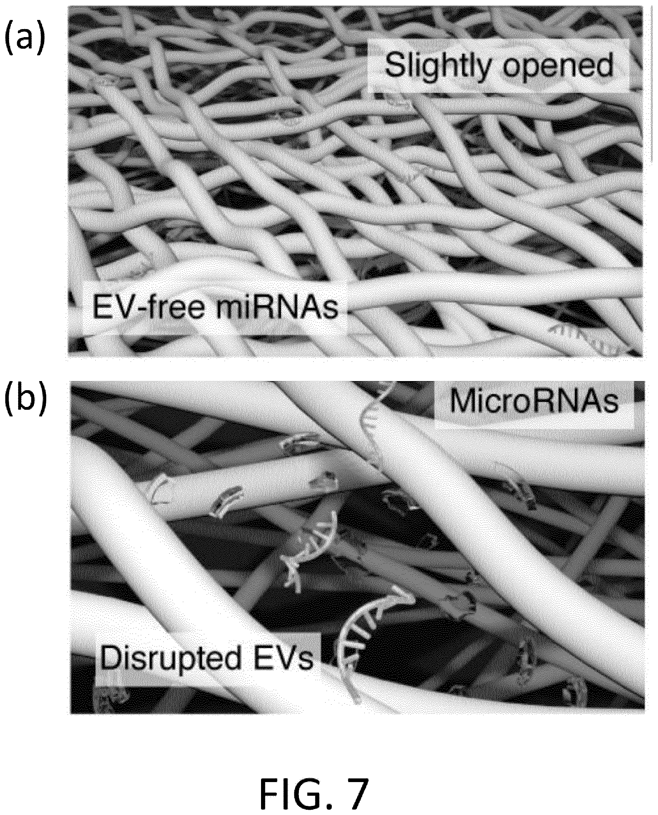

Fig. 7 shows, by computer graphics (CG), a step of using a cellulose nanofiber (CNF) sheet as a fiber sheet to disrupt extracellular vesicles captured as a target substance and extract miRNAs contained therein.

The extracellular vesicles (not shown) captured by the CNF sheet are washed under appropriate conditions. Thus, contaminants, for example, free miRNAs (EV-free miRNAs), can be washed out of the CNF sheet (Fig. 7 (a)). The CNF sheet is then immersed in a disrupting liquid (for example, a lysis buffer) to impregnate the CNF sheet with the disrupting liquid of the extracellular vesicles. The disrupting liquid can disrupt the extracellular vesicles and take out miRNAs inside the extracellular vesicles from the CNF sheet (Fig. 7 (b)).

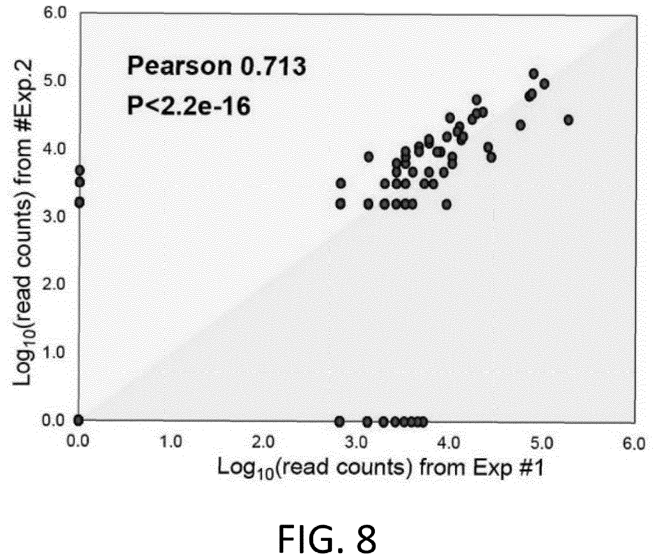

The profile of miRNAs in the EVs obtained by this method was confirmed to be relatively stable. Fig. 8 shows the correlation of miRNAs between two miRNA extractions using the method described above. Each dot represents one miRNA. The horizontal axis represents the read count in measurement #1, and the vertical axis represents the read count in measurement #2. The number of reads was determined with an RNA sequencer (MiSEQ, Illumina, Inc.). This shows reproducibility in the two extractions and measurements.

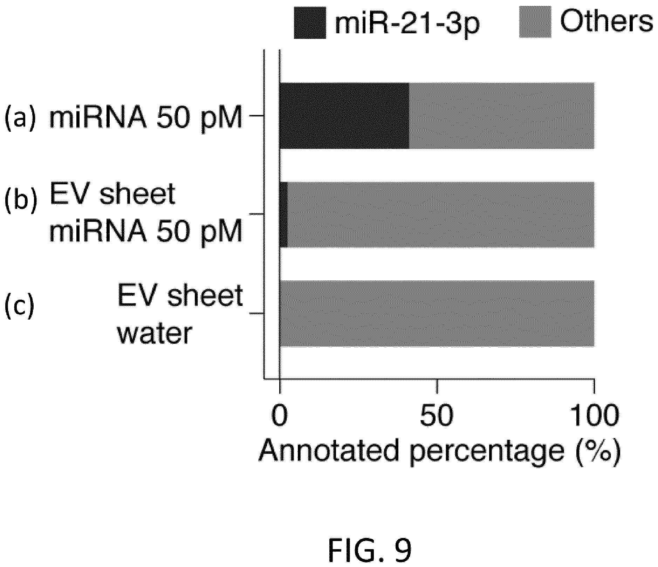

Due to the doubt that free miRNAs were being read, measurements were performed with synthetic miRNAs. More specifically, a 50 pM synthetic miR-21-3p solution was prepared. This was directly analyzed with the RNA sequencer (a). Furthermore, the solution was absorbed and captured by the CNF sheet in the produced state, was treated with a lysis buffer for 5 minutes, and was purified using a column. This solution was analyzed with the RNA sequencer (b). On the other hand, the CNF sheet was allowed to absorb water, the lysis buffer was introduced thereto to obtain a solution, and the solution was analyzed with the RNA sequencer (c). Fig. 9 shows a comparison of these annotation rates.

In the case of water alone, as a matter of course, no miR-21-3p was detected (c). The detected amount of miR-21-3p (annotation rate) in the synthetic miR-21-3p solution absorbed in the CNF sheet (b) was much lower than that in the solution directly measured (a). This shows that free miRNAs adsorbed in the CNF sheet barely affect the measurement results of miRNAs derived from EVs.

### <Example: Drying>

In the present example, 10 µL of the same sample as described above was dropped with a pipette on a 1 cm x 1 cm CNF sheet (hereinafter referred to as a fiber sheet). The fiber sheet was kept at room temperature and in the atmospheric environment in a laboratory for 3 days and 7 days. Thus, the fiber sheet was dried. The fiber sheet was then immersed in and washed with PBS. The fiber sheet was then immersed in 1.5 mL of a lysis buffer (Norgen) and was shaken with Voltex. 1 mL of the lysis buffer was taken out and purified using a Norgen kit to yield 50 µL of a miRNA solution. RNA in the solution was analyzed with the RNA sequencer (MiSEQ, Illumina, Inc.). The number of reads was 1.5 million to 2.3 million or more.

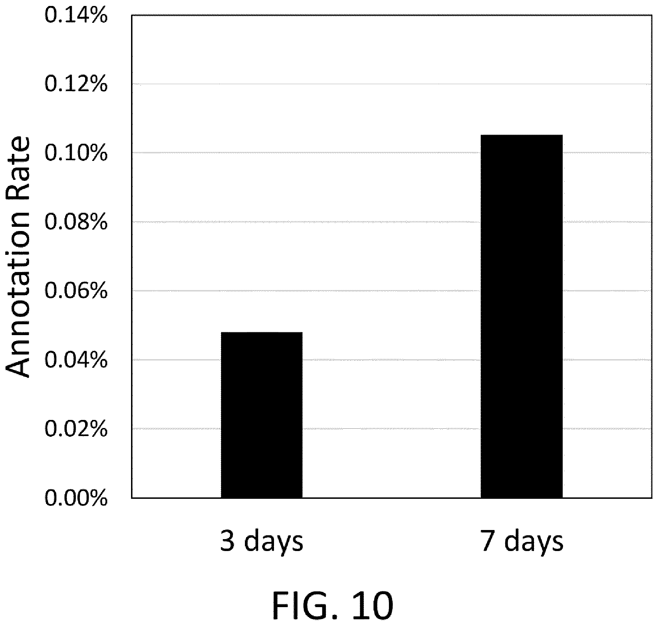

Fig. 10 shows the average values of the annotation rates obtained from a plurality of samples after drying for 3 days and drying for 7 days. The annotation rate ranged from approximately 0.03% to approximately 0.07% (approximately 0.05% on average) for drying for 3 days and from approximately 0.07% to approximately 0.14% (approximately 0.105% on average) for drying for 7 days. In this experiment, the annotation rate was higher for 7 days than for 3 days, suggesting that drying advanced in 7 days.

It was observed that the annotation rate did not change after 5, 6, or more days at standard atmospheric conditions in Japan. This shows that the fiber sheet is completely dried in 5 to 6 days. The degree of drying may be confirmed by another method. The speed and degree of drying can be further improved depending on the drying time, the humidity, and other conditions.

### <Example: EVs in Human Serum>

A method according to an embodiment of the present disclosure was used to absorb human serum into a fiber sheet and separate EVs in the human serum.

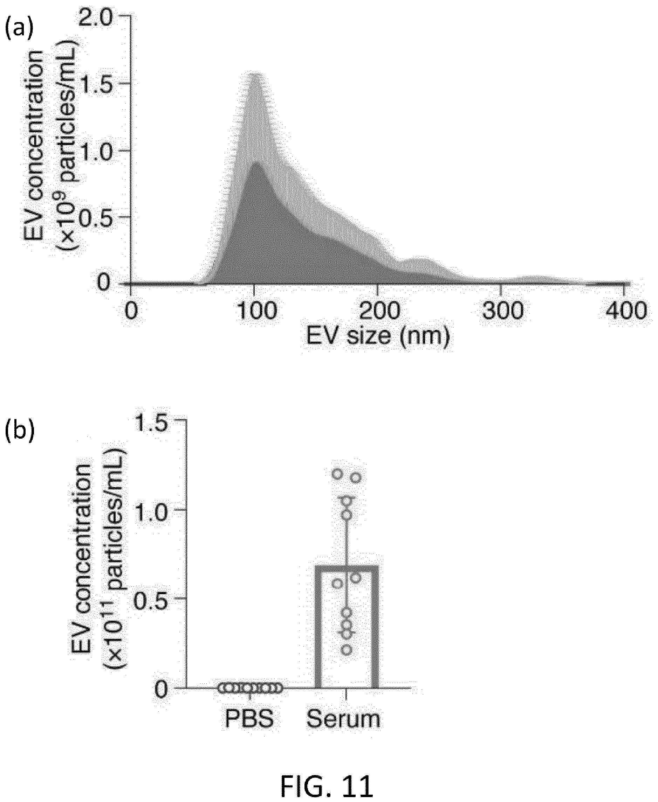

Fig. 11(a) shows the size distribution of EVs taken out from the fiber sheet. The EV size was measured with a nanoparticle analysis system (NanoSight, Malvern PANalyticalm). The horizontal axis represents the particle size (nm), and the vertical axis represents the concentration of particles (x 10⁹ /mL). Fig. 11(a) shows that particles obtained had a peak at approximately 105 nm and a distribution from approximately 60 nm to approximately 260 nm. It was presumed from this that the measured particles were EVs.

Furthermore, an unused fiber sheet was immersed in PBS for 10 seconds, and a solution obtained therefrom was also subjected to the same measurement. Unused fiber sheets contain dust inside (typically approximately 10⁸ /mL). Fig. 11(b) shows a comparison between the case of the unused fiber sheet unused (PBS, left side) and the case of Fig. 11(a). This shows that these particles are almost EVs and the influence of dust and the like is negligible.

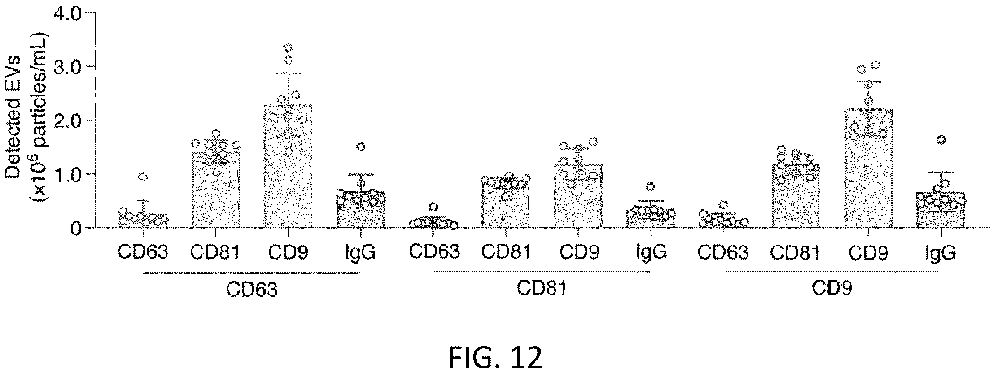

As shown in Fig. 12, specific proteins expressed in particles obtained in the same manner were detected with an exosome identification/quantification apparatus (Exoview Human Tetraspanin Kit, NanoView Bioscience). Anti-CD63 antibody, anti-CD9 antibody, and anti-CD81 antibody were used as primary antibodies. The detection of CD9, CD63, and CD81 showed that the obtained particles were EVs. The detection of CD63 and CD9 and less nonspecific adsorption to IgG show that the obtained particles were EVs.

### <Example: EVs in Ascites on Tumor Surface of Mouse Ovarian Cancer>

A method according to an embodiment of the present disclosure was used to attach a fiber (CNF) sheet to a tumor surface of a tumor-bearing mouse transplanted with ovarian cancer, absorb ascites, and separate EVs in the ascites.

A female athymic (NCr-nude) mouse was used. A mouse ovarian cancer cell line ID8 was intraperitoneally (IP) injected. A 14-day-old mouse was subjected to laparotomy, a fiber sheet was attached to the tumor surface, and the body fluid (ascites) on the surface was absorbed and obtained. In the case of the ID8 cell line, the ascites usually stops in 30 to 40 days and not in 14 days.

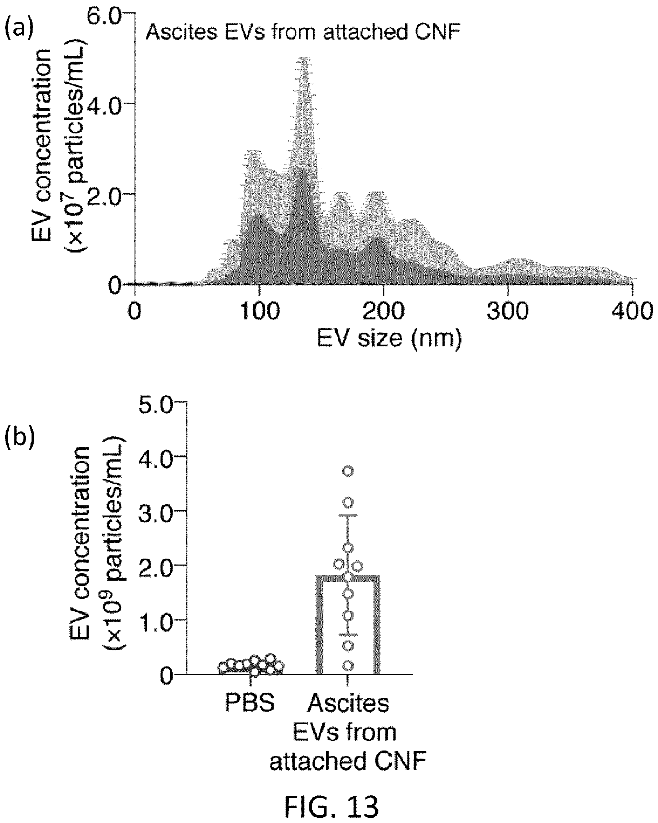

Fig. 13(a) shows the size distribution of EVs taken out from the fiber sheet. The EV size was measured with a nanoparticle analysis system (NanoSight, Malvern PANalyticalm). The horizontal axis represents the particle size (nm), and the vertical axis represents the concentration of particles (x 10⁷ /mL). Fig. 11(a) shows that particles obtained have a maximum peak at approximately 130 nm and a plurality of sub-peaks with other sizes and have a distribution from approximately 60 nm to approximately 400 nm. It was presumed from this that the measured particles were EVs.

Furthermore, an unused fiber sheet was immersed in PBS for 10 seconds, and a solution obtained therefrom was also subjected to the same measurement. Fig. 13(b) shows a comparison between the case of the unused fiber sheet unused (PBS, left side) and the case of Fig. 13(a). This shows that these particles are almost EVs and the influence of dust and the like is negligible.

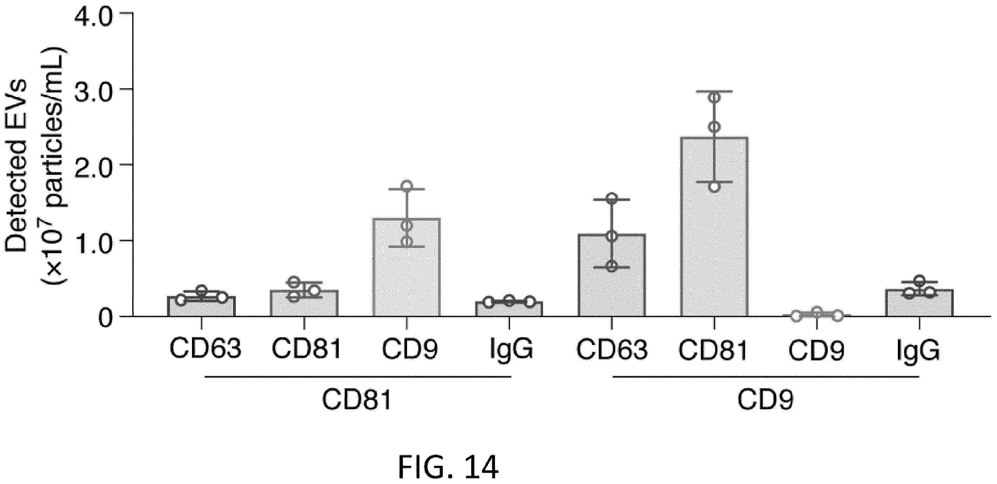

As shown in Fig. 14, specific proteins expressed in particles obtained in the same manner were detected with an exosome identification/quantification apparatus (Exoview Human Tetraspanin Kit, NanoView Bioscience). Anti-CD63 antibody, anti-CD9 antibody, and anti-CD81 antibody were used as primary antibodies. The detection of CD9, CD63, and CD81 showed that the obtained particles were EVs. The detection of CD63 and CD9 and less nonspecific adsorption to IgG show that the obtained particles were EVs.

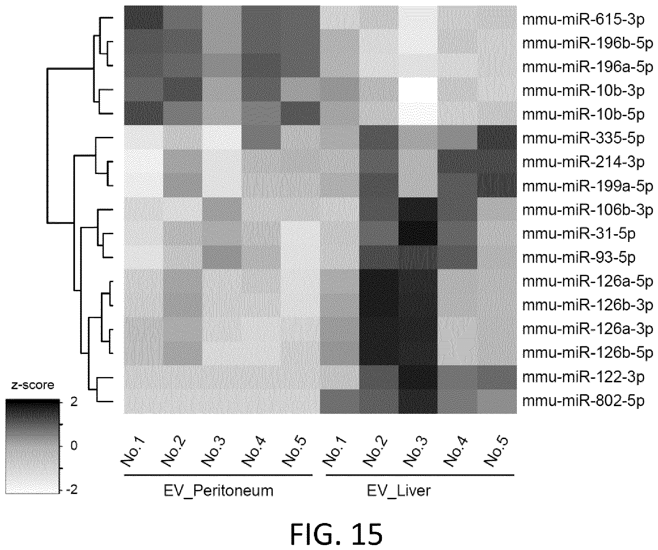

Fig. 15 shows a heat map of the expression of miRNAs obtained by RNA sequencing for 17 miRNAs contained in EVs taken out from the fiber sheet (liver surface (n = 5), peritoneal surface (n = 5)). Five miRNAs (mmu-miR-615-3p, mmu-miR-196b-5p, mmu-miR-196a-5p, mmu-miR-10b-3p, and mmu-miR-10b-5p) were more highly expressed in EVs from the pelvic peritoneum than those from the liver surface. On the other hand, 12 miRNAs (mmu-miR-335-5p, mmu-miR-214-3p, mmu-miR-199a-5p, mmu-miR-106b-3p, mmu-miR-31-5p, mmu-miR-93-5p, mmu-miR-126a-5p, mmu-miR-126b-3p, mmu-miR-126a-3p, mmu-miR-126b-5p, mmu-miR-122-3p, and mmu-miR-802-5p) were highly expressed in EVs from the liver surface. Thus, it was found that EV-containing miRNAs on the surface vary depending on the organ or the site in the body.

Samples were obtained from attachment of the fiber sheet to the tumor surface (Sheet-Tumor, Ev sheets T1 to T3), attachment of the fiber sheet to a normal tissue (Sheet-Normal, Ev sheets N1 to N3), tumor tissues themselves (Tissue-Tumor Tumor, Tissues T1 to T3), and normal tissues themselves (Tissue-Normal, Tissues N1 to N3, obtained by absorbing a trace amount of ascites of a normal mouse into the tissue sheet).

For the body fluid obtained with the fiber sheet, the fiber sheet was washed with PBS while EVs were captured, and the EVs were then disrupted. RNA in the solution was analyzed with the RNA sequencer (MiSEQ, Illumina, Inc.).

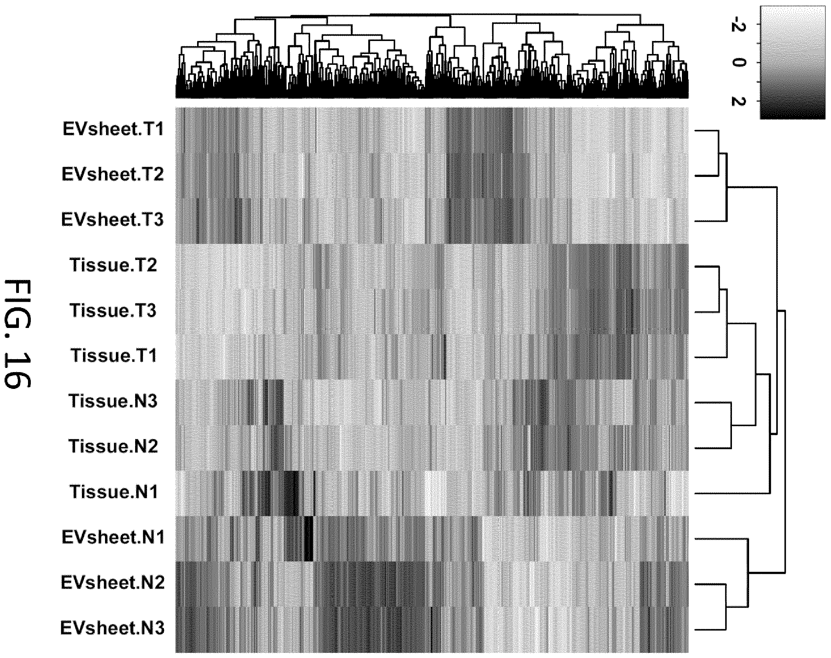

Fig. 16 shows a heat map of 254 miRNAs obtained by the RNA sequencing.
As can be seen, miRNAs in EVs obtained using the EV sheet had a different miRNA profile than the tissue. Furthermore, the ascites of a tumor-bearing mouse and the ascites of a non-tumor-bearing normal mouse showed completely different miRNA profiles.

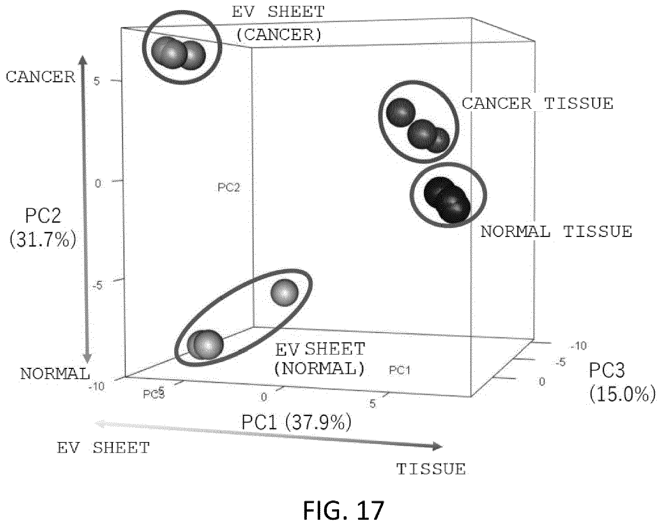

The detected miRNAs were subjected to principal component analysis (PCA). In Fig. 17, a first principal component (PC1) is a variable with "sheet" (-) and "tissue" (+), a second principal component (PC2) is a variable with "cancer" (-) and "normal" (+), and a third principal component (PC3) is a variable with "cancer" (-) and "normal" (+). Based on miRNAs obtained from the inside of the tissue, the cancer tissue and the normal tissue could be sufficiently clearly distinguished (PC2). On the other hand, it was found that the cancer tissue and the normal tissue can be more clearly distinguished based on the miRNAs obtained using the fiber sheet. As can be seen, miRNAs in EVs obtained using the EV sheet had a different miRNA profile than the tissue. Furthermore, the ascites of a tumor-bearing mouse and the ascites of a non-tumor-bearing normal mouse showed completely different miRNA profiles.

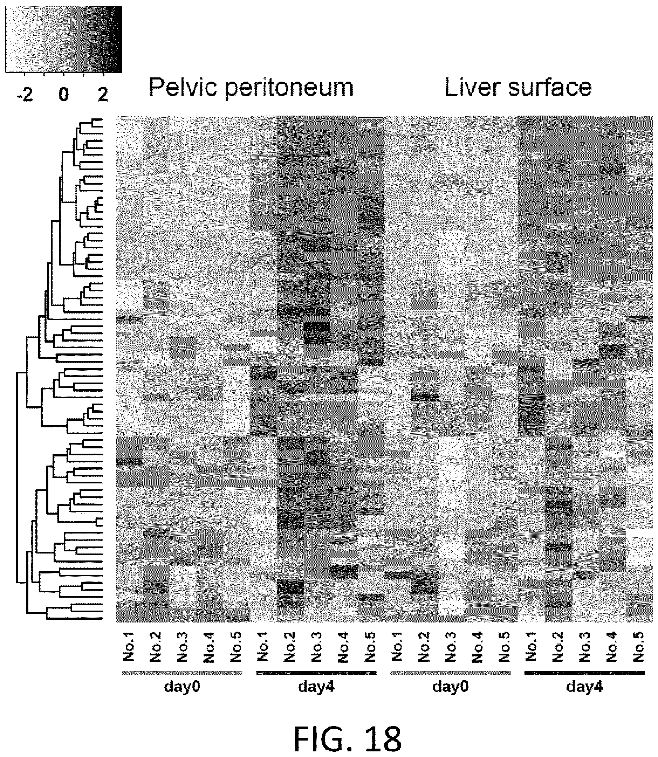

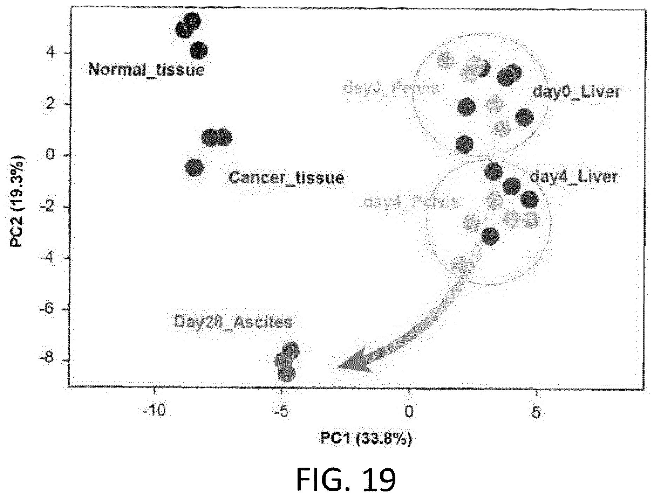

Fig. 18 is a heat map of the expression of miRNAs contained in EVs obtained using the fiber sheet from the pelvic peritoneum and the liver surface at day 0 and day 4 (n = 5 each). In this way, early cancer progression can be detected. Fig. 19 is a PCA map of the expression of miRNAs in EVs and tissues obtained using the fiber sheet. Changes from day 0 to day 4, that is, early cancer progression, were also observed on the PCA map. These changes on the PCA map also suggest that the cancer progression shifts toward the ascites at day 28. In this way, it is possible to attach the fiber sheet to obtain physiological EVs from a trace amount of body fluid and analyze very early cancer progression.

### <Example: EVs in Ascites on Tumor Surface of Human Ovarian Cancer>

A method according to an embodiment of the present disclosure was used to attach a fiber sheet to a tumor surface of a human ovarian cancer to absorb ascites and separate EVs in the ascites.

A fiber sheet was attached to a surface of a tumor excised from an ovarian cancer patient by laparotomy to absorb ascites (Surface), and a fiber sheet was immersed in ascites naturally flowing out from the excised tumor to absorb the ascites (Near). EVs captured therein were separated from each fiber sheet. While the EVs were captured, the fiber sheet was washed with PBS, and the EVs were then disrupted. miRNAs contained in each sample were analyzed with the RNA sequencer.

They had significantly different gene profiles from each other (not shown). It has been revealed that there is ascites derived from a tumor, which is different from a large amount of ascites that has been generally analyzed. Furthermore, it was revealed that EVs contained therein have different gene profiles.

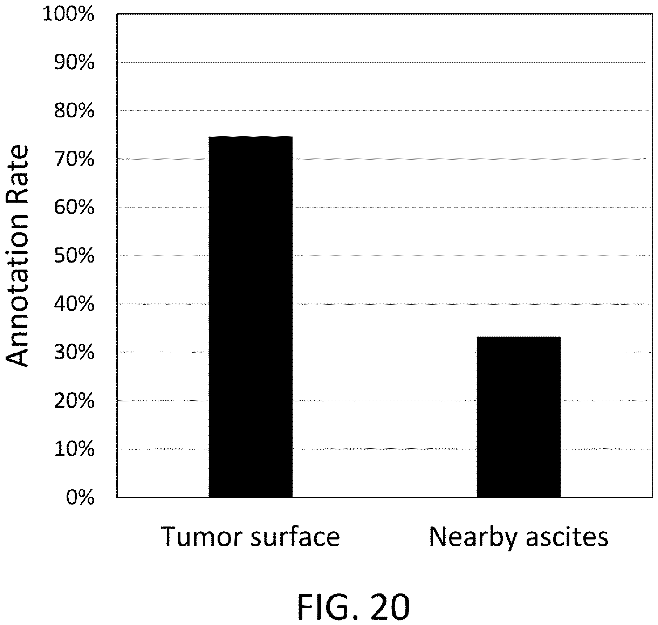

Fig. 20 shows the annotation rate in sequencing results obtained from each sample. The annotation rate of miRNA obtained from the tissue surface was approximately 75% and was very high. On the other hand, the annotation rate of miRNA obtained from the ascites naturally flowing out from the tissue was approximately 34%, which was lower than that of the fiber surface but was also very high. This is interpreted to be because a difference in annotation rate results from the influence of contamination with various nucleic acids between a sample containing a large number of EVs derived from the tumor and a body fluid affected by various humoral factors.

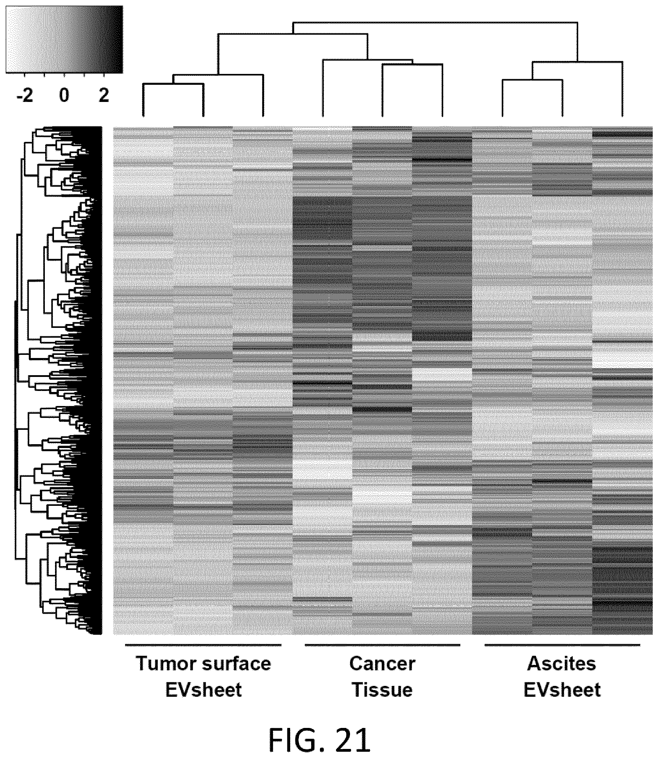

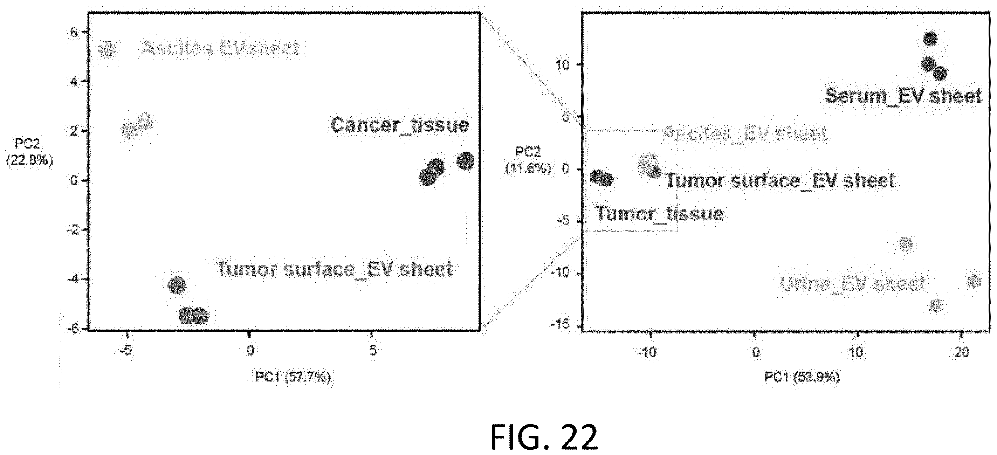

Fig. 21 shows a heat map of the expression of miRNAs obtained by the RNA sequencing for miRNAs from the EVs obtained using the fiber sheet and the tumor (n = 3, tumor surface-fiber sheet, ascites of tumor tissue-fiber sheet; and cancer tissue). It was found that the expression of miRNAs differed among them. Fig. 22 shows PCA maps of miRNAs obtained using tumor surface-fiber sheet; ascites-fiber sheet; tumor tissue; and serum-fiber sheet; and urine-fiber sheet (n = 3). Such clustering analysis also showed that the EVs on the tumor surface have a unique miRNA profile as compared with those of the tumor tissue and the ascites. The miRNA profile of the EVs on the tumor surface was also found to be closer to that of the tumor tissue than that of the EVs of the ascites (not shown). These results suggest that the EVs on the tumor surface were EVs released from the tumor and collected by the fiber sheet.

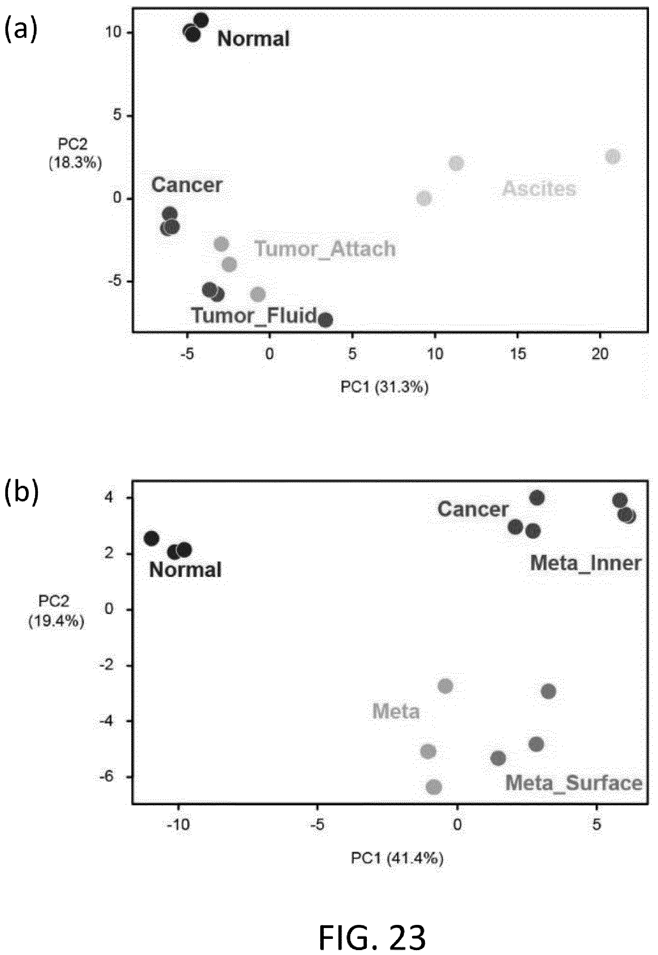

Figs. 23(A) and (B) are PCA maps of various data. The EVs of the ascites showed a different profile from the others. The tumor tissue and the tumor surface had similar profiles. Interestingly, EVs obtained by attaching the fiber sheet to the inside of a metastatic tumor (the surface exposed by opening the tumor) had a profile similar to a primary tumor tissue. On the other hand, the tissues of the primary tumor and the metastatic tumor had different profiles. Thus, using a fiber sheet, a new approach can reveal a previously unknown profile of a cancer of a patient. For example, based on any of the type, the amount, and the profile of a substance (RNA) in EVs obtained using a fiber sheet, it is possible to estimate the nature of the tissue (for example, metastatic, primary, or the like), the cancer type, the degree of progress of the cancer, and the like.

In some embodiments, the fiber sheet may be attached to a non-invasively accessible site (for example, skin, mucosa) or the like to collect biomolecules, such as EVs. In some embodiments, the fiber sheet may be invasively attached to a site in the body to collect biomolecules, such as EVs. For example, a site within the body may be accessed by surgical means. The surgical means is, for example, but not limited to, laparotomy, laparoscopic surgery, or the like. In one operation, a fiber sheet may be attached to a plurality of organs or sites to collect biomolecules, such as EVs, from each organ or site. It may be possible to determine the amount, type, profile, or the like of a biomolecule (for example, EV) collected by a fiber sheet or a substance (for example, a nucleic acid, such as DNA or RNA, a protein, or the like) contained in the biomolecule. It may be possible to generate information usable for diagnosis. For example, the type/subtype of a disease, the stage/degree of progress of a disease, the localization or diffuseness of a disease, or the like may be estimated, and a prediction or a proposal of a strategy, such as diagnosis, prognostication, or prevention, can be made on the basis thereof.

The present disclosure also includes the following embodiments:
A001 A method for preserving a target substance in a target liquid, the method including:
   providing a fiber sheet;
   bringing the fiber sheet into contact with the target liquid to absorb the target liquid into the fiber sheet;
   drying the fiber sheet; and
   washing the fiber sheet.
A001b A method for preserving a target substance in a target liquid, the method including:
   providing a fiber sheet;
   bringing the fiber sheet into contact with the target liquid to absorb the target liquid into the fiber sheet; and
   drying the fiber sheet.
A002 A method for separating a target substance in a target liquid, the method including:
   providing a fiber sheet;
   bringing the fiber sheet into contact with the target liquid to absorb the target liquid into the fiber sheet;
   drying the fiber sheet;
   washing the fiber sheet; and
   applying an aqueous solution to the dried fiber sheet to release a captured target substance from the fiber sheet.
A003 A method for separating a target substance in a target liquid, the method including:
   providing a fiber sheet;
   bringing the fiber sheet into contact with the target liquid to absorb the target liquid into the fiber sheet;
   drying the fiber sheet;
   washing the fiber sheet; and
   disrupting the target substance captured by the fiber sheet to obtain an internal substance.
A011 The method according to any one of the embodiments A001 to A003 or any embodiment, wherein
   the fiber sheet is substantially configured so that
   the target substance can enter the fiber sheet when the target liquid is absorbed, and
   the target substance is retained in the fiber sheet during drying the fiber sheet and washing the fiber sheet.
A021 The method according to any one of the embodiments A001 to A011 or any embodiment, wherein
   the target substance is an extracellular vesicle (EV).
A022 The method according to the embodiment A021 or any embodiment, wherein
   the fiber sheet substantially has a pore size of 40 nm to 500 nm.
A023 The method according to the embodiment A022 or any embodiment, wherein
   the fiber sheet substantially has a pore size of 40 nm to 200 nm.
A024 The method according to the embodiment A022 or any embodiment, wherein
   the fiber sheet substantially has a pore size of 200 nm to 500 nm.
A025 The method according to any one of the embodiments A001 to A024 or any embodiment, wherein
   the target substance is a virus.
A026 The method according to the embodiment A025 or any embodiment, wherein
   the fiber sheet substantially has a pore size of 20 nm to 500 nm.
A031 The method according to any one of the embodiments A001 to A026 or any embodiment, wherein
   the target liquid is a body fluid.
A041 The method according to any one of the embodiments A001 to A026 or any embodiment, wherein
   the bringing of the fiber sheet into contact with the target liquid includes
   bringing at least one surface of the fiber sheet into contact with a target object containing the target liquid.
A042 The method according to the embodiment A041 or any embodiment, wherein
   the target object containing the target liquid is at least one selected from the group consisting of a skin, a mucosa, an organ, and a tumor.
A043 The method according to the embodiment A041 or A042 or any embodiment, wherein
   the bringing of the fiber sheet into contact with the target liquid includes attaching the fiber sheet to a surface of the target object to allow the fiber sheet to absorb a body fluid present on the surface of the target object.
A051 The method according to any one of the embodiments A001 to A043 or any embodiment, wherein
   the drying of the fiber sheet is performed at room temperature.
A052 The method according to any one of the embodiments A001 to A051 or any embodiment, wherein
   the drying of the fiber sheet is performed in an air atmosphere or in a vacuum atmosphere.
A053 The method according to any one of the embodiments A001 to A052 or any embodiment, wherein
   the drying of the fiber sheet includes reducing the pore size.
A061 The method according to any one of the embodiments A001 to A053 or any embodiment, wherein
   the washing of the fiber sheet includes washing the fiber sheet such that the target substance target is retained by the fiber sheet and/or does not flow out to an outside.

While preferred embodiments of the present invention have been shown and described herein, it will be apparent to those skilled in the art that such embodiments are provided by way of example only. It is not intended that the present invention be limited by the specific examples provided in the description. While the present invention has been described with reference to the description, the explanation and illustration of the embodiments herein are not meant to be construed in a limiting sense. Those skilled in the art will come up with numerous variations, changes, and substitutions without departing from the present invention. Furthermore, it is to be understood that all aspects of the present invention are not limited to the specific depictions, configurations, or relative proportions set forth herein that depend on various conditions and variables. It should be understood that various alternatives to the embodiments of the present invention described herein may be employed in practicing the present invention. Thus, it is intended that the present invention cover such alternatives, modifications, variations, or equivalents. It is intended that the claims of the present application define the scope of the present invention and that methods and structures within the scope of these claims and their equivalents be covered thereby.

## Claims

1. A method for preserving a target substance in a target liquid, the method comprising:
providing a fiber sheet;
bringing the fiber sheet into contact with the target liquid to absorb the target liquid into the fiber sheet;
drying the fiber sheet; and
washing the fiber sheet.

2. A method for separating a target substance in a target liquid, the method comprising:
providing a fiber sheet;
bringing the fiber sheet into contact with the target liquid to absorb the target liquid into the fiber sheet;
drying the fiber sheet;
washing the fiber sheet; and
applying an aqueous solution to the dried fiber sheet to release a captured target substance from the fiber sheet.

3. A method for separating a target substance in a target liquid, the method comprising:
providing a fiber sheet;
bringing the fiber sheet into contact with the target liquid to absorb the target liquid into the fiber sheet;
drying the fiber sheet;
washing the fiber sheet; and
disrupting the target substance captured by the fiber sheet to obtain an internal substance.

4. The method according to any one of Claims 1 to 3, wherein
the fiber sheet is substantially configured so that
the target substance can enter the fiber sheet when the target liquid is absorbed, and
the target substance is retained in the fiber sheet during drying the fiber sheet and washing the fiber sheet.

5. The method according Claims 1 to 3, wherein
the target substance is an extracellular vesicle (EV).

6. The method according to Claim 5, wherein
the fiber sheet substantially has a pore size of 40 nm to 500 nm.

7. The method according to Claim 6, wherein
the fiber sheet substantially has a pore size of 40 nm to 200 nm.

8. The method according to Claim 7, wherein
the fiber sheet substantially has a pore size of 200 nm to 500 nm.

9. The method according to any one of Claims 1 to 3, wherein
the target substance is a virus.

10. The method according to any one of Claims 1 to 3, wherein
the target liquid is a body fluid.

11. The method according to Claim 10, wherein
the bringing of the fiber sheet into contact with the target liquid includes
bringing at least one surface of the fiber sheet into contact with a target object containing the target liquid.

12. The method according to Claim 11, wherein
the target object containing the target liquid is at least one selected from the group consisting of a skin, a mucosa, an organ, and a tumor.

13. The method according to Claim 11 or 12, wherein
the bringing of the fiber sheet into contact with the target liquid includes attaching the fiber sheet to a surface of the target object to allow the fiber sheet to absorb a body fluid present on the surface of the target object.

14. The method according to any one of Claims 1 to 3, wherein
the drying of the fiber sheet is performed at room temperature.

15. The method according to any one of Claims 1 to 3, wherein
the drying of the fiber sheet is performed in an air atmosphere or in a vacuum atmosphere.

16. The method according to any one of Claims 1 to 3, wherein
the drying of the fiber sheet includes reducing the pore size.

17. The method according to any one of Claims 1 to 3, wherein
the washing of the fiber sheet includes washing the fiber sheet such that the target substance target is retained by the fiber sheet and/or does not flow out to an outside.
